# EUROPEAN PATENT SPECIFICATION

(11) **EP 2 323 493 B1**
(45) Date of publication and mention of the grant of the patent: **24.10.2012**
(21) Application number: 09815248.1
(22) Date of filing: 18.09.2009
(51) Int. Cl.: A23C 9/00, C12N 1/20

(54) **PROBIOTIC INFANT PRODUCTS**
PROBIOTISCHE SÄUGLINGSPRODUKTE
PROBIOTIQUES POUR BÉBÉS

(30) Priority: 19.09.2008 US 284208
(43) Date of publication of application: 25.05.2011
(73) Proprietor: Mead Johnson Nutrition Company, Evansville, IN 47721-0001 (US)
(72) Inventor: RUSSELL, William Michael,, Evansville, Indiana 47721-0001 (US); CEDDIA, Michael,, Evansville, Indiana 47721-0001 (US)
(74) Representative: Jansen, Cornelis Marinus
(86) International application number: PCT/US2009/057434
(87) International publication number: WO 2010/033775

(56) References cited:
- WO-A2-2008/117266
- US-A1- 2007 110 849
- US-A1- 2007 141 039
- NAKAMURA Y ET AL: "Agent for preventing and/or treating allergies, comprises Bifidobacterium, or its processed substance chosen from e.g. culture, concentrate and paste of Bifidobacterium, as an active ingredient", 20061012, vol. 2006, no. 80, 12 October 2006 (2006-10-12), XP002478986,
- CHOURAQUI ET AL.: 'Assessment of the safety, tolerance, and protective effect against diarrhea of infant formulas containing mixtures of probiotics or probiotics and prebiotics in a randomized controlled trial.' AM J CLIN NUTR. vol. 87, no. 5, May 2008, pages 1365 - 1373, XP008143235
- MEILE ET AL.: 'Safety assessment of dairy microorganisms: Propionibacterium and Bifidobacterium.' INT J FOOD MICROBIOL. vol. 126, no. 3, 01 September 2008, pages 316 - 320, XP025432125

## Description

### TECHNICAL FIELD

The present invention relates generally to probiotic infant formulas and children's nutritional products.

### DISCLOSURE OF THE INVENTION

Briefly, the present invention is directed, in an embodiment, to an infant formula or children's nutritional product comprising a protein source, a fat source, a carbohydrate source, and *B. longum* AH1206.

In another embodiment, the present invention is directed to a method for reducing inflammation in an infant or child comprising administering *B. longum* AH1206 to the infant or child.

### BRIEF DESCRIPTION OF THE DRAWINGS

For a more complete understanding of the present invention, reference is now made to the following descriptions taken in conjunction with the accompanying drawings.

Fig. 1 is a BOX polymer chain reaction (BOX PCR) (bioanalyser) barcode profile for *B. longum* AH1206. Base pair sizes were determined using the Agilent 2100 software;

Fig. 2 is a graph illustrating the fecal recovery of *B. longum* AH1206 over an 8 day feeding period and demonstrates that AH1206 can survive the murine gastrointestinal tract;

Fig. 3 is a bar graph showing the effect of *B. longum* AH1206 on interleukin (IL)-10 cytokine production by human peripheral blood mononuclear cells (PBMCs). Results are expressed as mean ± standard error of measurement (SEM) (n=6);

Fig. 4 is a bar graph showing the effect of *B. longum* AH1206 feeding on eosinophil recruitment to the lungs of sensitized mice. Fig. 4A shows that the total number of cells present in bronchoalveolar lavage (BAL) were reduced in AH1206-fed mice; Fig. 4B shows that differential cell counts on BAL revealed that the reduction in cell numbers was primarily in the eosinophil population. (Cell number is expressed on the y-axis (x10⁴); *p<0.05 versus placebo);

Fig. 5A and B are graphs showing the effect of (A) probiotic bacterial strain AH1206 and (B) placebo on total cell numbers in BAL fluid following ovalbumin (OVA) challenge in sensitized animals (n=10/group, * = p<0.05 compared to OVA challenge alone);

Fig. 6A and B are graphs showing the effect of (A) *B. longum* AH1206 treatment and (B) placebo on airway responsiveness to methacholine, as assessed by changes in enhanced pause (Penh) in OVA-sensitized mice 24 hours after intranasal challenge with OVA or saline. Each data point represents the mean ± SEM (n=10/groups * p = <0.05 compared to OVA alone);

Fig. 7 is a graph showing the tumor necrosis factor (TNF) cytokine level in BAL fluid from OVA-sensitized mice. Each column represents the mean ± SEM (n=10, * p<0.05 compared to OVA-challenged, MRS (deMann, Rogosa and Sharpe) broth-treated control);

Fig. 8A and B are graphs showing the effect of oral treatment with probiotic strain AH1206 on (A) TNF and (B) interferon (IFN)-γ cytokine production from activated splenocytes isolated from OVA-sensitized mice (CD3/CD28 stimulated splenocytes). Each column represents the mean ± SEM (n=10, * p=<0.05 compared to OVA-challenged, MRS broth-treated control);

Fig. 9 is a graph showing that the levels of OVA-specific immunoglobulin E (IgE) in serum isolated from mice fed AH1206 was significantly lower than the non-AH1206-fed controls (**p = <0.01);

Fig. 10 is a graph illustrating the effect of oral treatment of probiotic strain AH1206 on TNF-α production from activated splenocytes isolated from OVA-sensitized mice (CD3/CD28 stimulated splenocytes). The mean is illustrated for each group (* p = <0.05, ** p =<0.01 compared to OVA- and cholera toxin (CT)-challenged, MRS broth-treated control);

Fig. 11 is a graph illustrating that CD4⁺ CD25⁺ cells from AH1206 fed animals substantially reduced proliferation (n=10 for all groups except the control, in which n=20);

Fig. 12A and B are graphs showing the percentage of cells in the CD4⁺ population that are also CD25⁺, as assessed by flow cytometry (n=11 for the unfed group, n=20 for placebo group, and n=10 for the AH1206 fed group);

Fig. 13 illustrates the percentage of CD4/CD25⁺ cells expressing the transcription factor FoxP3 is significantly upregulated in germ-free mice consuming AH1206 (n = 8 or 9 per group) (*p<0.05 v. placebo); and

Fig. 14 is a graph illustrating the stability of probiotic strain AH1206 over 3 months.

### BEST MODE FOR CARRYING OUT THE INVENTION

Reference now will be made in detail to the embodiments of the invention, one or more examples of which are set forth below. Each example is provided by way of explanation of the invention, not a limitation of the invention. In fact, it will be apparent to those skilled in the art that various modifications and variations can be made in the present invention without departing from the scope or spirit of the invention. For instance, features illustrated or described as part of one embodiment, can be used on another embodiment to yield a still further embodiment.

Thus, it is intended that the present invention covers such modifications and variations as come within the scope of the appended claims and their equivalents. Other objects, features and aspects of the present invention are disclosed in or are obvious from the following detailed description. It is to be understood by one of ordinary skill in the art that the present discussion is a description of exemplary embodiments only, and is not intended as limiting the broader aspects of the present invention.

As set forth above, the present invention relates generally to probiotic infant formulas and children's nutritional products. References related to probiotic infant formulas and children's nutritional products may include U.S. Patent Nos. 7,179,460 to Dennen, et al. and 5,922,375 to Luchansky, et al.

The technical problem to be solved by the present invention is to provide infant formulas and children's nutritional products containing novel probiotics. Thus, in an embodiment, the present invention is directed to an infant formula or children's nutritional product containing *Bifidobacterium longum* strain AH1206. A deposit of B. *longum* strain AH1206 was made at the National Collections of Industrial, Marine, and Food Bacteria (NCIMB), Scotland, UK, on May 11, 2006 and was accorded the accession number NCIMB 41382. The NCIMB is an International Depositary Authority recognized under the Budapest Treaty. The strain is acid and bile tolerant and transits the gastrointestinal tract. In a particular embodiment of the invention, *B. longum* AH1206 is isolated from infant feces.

In another embodiment, the infant formula or children's nutritional product contains *B. longum* strain AH1206 or a mutant or variant thereof. The mutant may be a genetically modified mutant. The variant may be a naturally occurring variant of *Bifidobacterium.* In some embodiments, the strain may be a probiotic. In other embodiments, the strain may be in the form of a biologically pure culture. In still other embodiments, the strain is an isolated strain.

As used herein, the terms "mutant", "variant", and "genetically modified mutant" include a strain of Bifidobacteria whose genetic and/or phenotypic properties are altered compared to the parent strain. Naturally occurring variants of *Bifidobacterium longum* include the spontaneous alterations of targeted properties selectively isolated. Deliberate alteration of parent strain properties is accomplished by conventional (in vitro) genetic manipulation technologies, such as gene disruption and conjugative transfer.

Genetic modification includes introduction of exogenous and/or endogenous DNA sequences into the genome of a Bifidobacteria strain, for example by insertion into the genome of the bacterial strain by vectors, including plasmid DNA, or bacteriophages. Natural or induced mutations include at least single base alterations such as deletion, insertion, transversion or other DNA modifications which may result in alteration of the amino acid sequence encoded by the DNA sequence.

The terms "mutant", "variant", and "genetically modified mutant" also include a strain of Bifidobacteria that has undergone genetic alterations that accumulate in a genome at a rate which is consistent in nature for all micro-organisms and/or genetic alterations which occur through spontaneous mutation and/or acquisition of genes and/or loss of genes which is not achieved by deliberate (in vitro) manipulation of the genome but is achieved through the natural selection of variants and/or mutants that provide a selective advantage to support the survival of the bacterium when exposed to environmental pressures such as antibiotics. A mutant can be created by the deliberate (in vitro) insertion of specific genes into the genome which do not fundamentally alter the biochemical functionality of the organism, but whose products can be used for identification or selection of the bacterium, for example antibiotic resistance.

A person skilled in the art appreciates that mutant or variant strains of Bifidobacteria can be identified by DNA sequence homology analysis with the parent strain. Strains of Bifidobacteria having a close sequence identity with the parent strain are considered to be mutant or variant strains. A Bifidobacteria strain with a sequence identity (homology) of 96% or more, such as 97% or more, or 98% or more, or 99% or more with the parent DNA sequence may be considered to be a mutant or variant. Sequence homology may be determined using the on-line homology algorithm "BLAST" program, publicly available at http://www.ncbi.nlm.nih,gov/BLAST/.

Mutants of the parent strain also include derived Bifidobacteria strains having at least 85% sequence homology, such as at least 90% sequence homology, or at least 95% sequence homology to the 16s-23s intergenic spacer polynucleotide sequence of the parent strain. These mutants may further comprise DNA mutations in other DNA sequences in the bacterial genome.

In one embodiment of the invention, the *Bifidobacterium* strain is in the form of viable cells. Alternatively, the *Bifidobacterium* strain may be in the form of inactivated cells. The term "inactivated" means that the metabolic activity or reproductive ability of the strain has been reduced or destroyed. Inactivation may occur through any method currently known in the art or yet to be developed. The inactivation may be accomplished, for example, via heat treatment, lyophilization, ultraviolet light, gamma radiation, pressure, chemical disruption, mechanical disruption, or alteration of pH. For example, the probiotic may be inactivated with heat treatment via storage in the range of 80°C to 100°C for 10 minutes. The probiotic may also be inactivated with ultraviolet light via irradiation for 5 minutes at a distance of 5 cm from a 30 Watt UVC lamp. Alternatively, the probiotic may be inactivated with gamma radiation via irradiation with 2 kg-Gray (kGy) using a Cobalt-60 source at a distance of 20 cm. As used herein, the term "inactivated" is synonymous with "non-viable".

In an embodiment, *B. longum* strain AH1206 may be supplemented into a formulation for children or infants. The term "infant" means a postnatal human that is less than about 1 year old. The term "child" means a human in the age range of about 1 and 12 years old. In certain embodiments, a child is in the age range of about 1 and 6 years old. In other embodiments, a child is in the age range of about 7 and 12 years old.

The amount of *B. longum* AH1206 supplemented into the formulation of the invention may correspond to the range of about 1x10⁴ to about 1x10¹² colony forming units (cfu) per gram formulation. In another embodiment, the amount supplemented into the formulation of the invention may correspond to the range of about 1x10⁶ and about 1x10⁹ cfu per gram formulation. In another embodiment, the amount supplemented into the formulation of the invention may correspond to the range of about 1x10⁹ and about 1x10¹² cfu per gram formulation. In another embodiment, the amount supplemented into the formulation of the invention may correspond to at least about 1x10⁶ cfu per gram formulation.

The form of administration of *B. longum* AH1206 in the present invention is not critical, as long as an effective amount is administered to a child or infant. In some embodiments, *B. longum* AH1206 is administered to a child or infant via tablets, pills, encapsulations, caplets, gelcaps, capsules, oil drops, sachets, liquids, liquid concentrates, powders, elixirs, solutions, suspensions, emulsions, lozenges, beads, and combinations thereof. In another embodiment, *B. longum* AH1206 is encapsulated in a sugar, fat, or polysaccharide. In yet another embodiment, *B. longum* AH1206 is added to a food or drink product and consumed. The food or drink product may be a nutritional supplement or a children's nutritional product such as a follow-on formula, growing up milk, beverage, milk, yogurt, fruit juice, fruit-based drink, chewable tablet, cookie, cracker, or a milk powder.

In other embodiments, the product may be an infant's nutritional product, such as an infant formula or a human milk fortifier. As used herein, the term "infant formula" means a composition that satisfies the nutrient requirements of an infant by being a substitute for human milk. The term "human milk fortifier" means a composition which can be added to human milk to enhance the nutritional value of the human milk. In some embodiments, the composition is an acidified product (as required by certain medical food regulations).

The nutritional product may be a product for a full-term infant, a preterm infant, a low-birth-weight infant, or a very-low-birth-weight infant. As used herein, the terms "preterm" or "preterm infant" may include low-birth-weight infants or very-low-birth weight infants. Low-birth-weight infants are those born from about 32 to about 37 weeks of gestation or weighing from about 3.25 to about 5.5 pounds at birth. Very-low-birth-weight infants are those born before about 32 weeks of gestation or weighing less than about 3.25 pounds at birth. Thus, preterm infants may include infants born before about 37 weeks gestation and/or those weighing less than about 5.5 pounds at birth.

In certain embodiments, the formulations may be administered enterally or parenterally. As used herein, "enteral" means through or within the gastrointestinal, or digestive, tract, and "enteral administration" includes oral feeding, intragastric feeding, transpyloric administration, or any other introduction into the digestive tract. The term "parenterally" means taken into the body or administered in a manner other than through the digestive tract, such as by intravenous or intramuscular injection.

The formulations of the invention may provide minimal, partial, or total nutritional support. The compositions may be nutritional supplements or meal replacements. In some embodiments, the compositions may be administered in conjunction with a food or nutritional composition. In this embodiment, the compositions can either be intermixed with the food or nutritional composition prior to ingestion by the subject or can be administered to the subject either before or after ingestion of a food or nutritional composition. The compositions may be administered to preterm infants receiving infant formula, breast milk, a human milk fortifier, or combinations thereof. The compositions may, but need not, be nutritionally complete. By the term "nutritionally complete," it is meant that the composition contains adequate nutrients to sustain healthy human life for extended periods.

In some embodiments, the invention may comprise a prenatal dietary supplement to be consumed by a pregnant woman, thereby providing *B. longum* AH1206 to the fetus in utero. In other embodiments, the invention may comprise a postnatal dietary supplement to be consumed by a nursing mother, thereby providing B. *longum* AH1206 to the postnatal infant via mother's milk.

If *B. longum* AH1206 is administered via an infant formula or children's nutritional product, the formulation may be nutritionally complete and contain suitable types and amounts of lipid, carbohydrate, protein, vitamins and minerals. The amount of lipid or fat typically may vary from about 3 to about 7 g/100 kcal. Lipid sources may be any known or used in the art, e.g., milk fat, egg yolk lipid, fish oil, vegetable oils such as palm oil, soybean oil, palmolein, palm oil, palm kernel oil, coconut oil, medium chain triglyceride oil, high oleic sunflower oil, olive oil, high oleic safflower oil, and esters of fatty acids.

The amount of protein typically may vary from about 1 to about 5 g/100 kcal. Protein sources may be any known or used in the art, e.g., milk protein, non-fat milk solids, nonfat milk, whey protein, casein, soy protein, animal protein, cereal protein, vegetable protein, or combinations thereof. The formulation may contain proteins and/or peptides rich in glutamine/glutamate. The protein source may be intact, partially hydrolyzed, or extensively hydrolyzed. The protein source, in some embodiments, may be a combination of intact protein and hydrolyzed protein. The protein source may be an isolate or a concentrate.

In certain embodiments, the composition of the invention may contain a nitrogen source (i.e., amino acids and/or protein) such that the total amount of amino acids or protein may be from about 1 g/100 kilocalories (kcal) to about 10 g/100 kcal of total composition, in some embodiments about 2 g/100 kcal to about 6 g/100 kcal. The amount of lipid source per 100 kcal of total composition may be greater than 0 g up to about 6 g, in some embodiments about 0.5 g to about 5.5 g, and in other embodiments about 2 g to about 5.5 g; and the amount of non-fiber carbohydrate source per 100 kcal of total composition may be about 5 g to about 20 g, and in some embodiments may be about 7.5 g to about 15 g. The amount of vitamins and minerals in the nutritionally complete composition may be sufficient to meet 100% of the U.S. recommended daily intake (RDI) of about 500 to about 3,000 kcal, in some embodiments about 1,000 to about 3,000 kcal. In a particular embodiment, the composition may be protein-free. In such an embodiment, the composition may contain a protein equivalent source that comprises 100% free amino acids.

The amount of carbohydrate typically may vary from about 8 to about 12 g/100 kcal. Carbohydrate sources may be any known or used in the art, e.g., lactose, fructose, glucose, corn syrup, corn syrup solids, maltodextrins, sucrose, starch, rice syrup solids, rice starch, modified corn starch, modified tapioca starch, rice flour, soy flour, and combinations thereof. The formulation may include any one or more of an adjuvant, a bacterial component, a drug entity, or a biological compound.

Conveniently, commercially available infant formulas and other formulations may be used in practice of the present invention. For example, Enfamil®, Enfamil® Premature Formula, Enfamil® with Iron, Lactofree®, Nutramigen®, Pregestimil®, and ProSobee® (available from Mead Johnson & Company, Evansville, IN, U.S.A.) may be supplemented with suitable levels of *B. Iongum* AH1206 and used in practice of the invention.

The formulation of the present invention may optionally include one or more of the following vitamins or derivatives thereof, including, but not limited to, biotin, vitamin B₁, thiamin, thiamin pyrophosphate, vitamin B₂, riboflavin, flavin mononucleoride, flavin adenine dinucleotide, pyridoxine hydrochloride, thiamin mononitrate, folic acid, vitamin B₃, niacin, nicotinic acid, nicotinamide, niacinamide, nicotinamide adenine dinucleotide, tryptophan, biotin, pantothenic acid, vitamin B₆, vitamin B₁₂, cobalamin, methylcobalamin, deoxyadenosylcobalamin, cyanocobalamin, calcium pantothenate, pantothenic acid, vitamin C, ascorbic acid, vitamin A, retinol, retinal, retinoic acid, beta-carotene, vitamin D, vitamin D₃, calciferol, cholecalciferol, dihydroxy vitamin D, 1,25-dihydroxycholecalciferol, 7-dehyrdocholesterol, choline, vitamin E, vitamin E acetate, vitamin K, menadione, menaquinone, phylloquinone, naphthoquinone, and mixtures thereof.

The formulation of the present invention may optionally include one or more of the following minerals or derivatives thereof, including, but not limited to, phosphorus, potassium, sulfur, sodium, docusate sodium, chloride, manganese, magnesium, magnesium stearate, magnesium carbonate, magnesium oxide, magnesium hydroxide, magnesium sulfate, copper, cupric sulfate, iodide, boron, zinc, zinc oxide, chromium, molybdenum, iron, carbonyl iron, ferric iron, ferrous fumarate, polysaccharide iron, fluoride, selenium, molybdenum, calcium phosphate or acetate, potassium phosphate, magnesium sulfate or oxide, sodium chloride, potassium chloride or acetate, ferric orthophosphate, alpha-tocopheryl acetate, zinc sulfate or oxide, copper gluconate, chromium chloride or picolonate, potassium iodide, sodium selenate, sodium molybdate, phylloquinone, cyanocobalamin, sodium selenite, copper sulfate, inositol, potassium iodide, cobalt, and mixtures thereof. Non-limiting exemplary derivatives of mineral compounds include salts, alkaline salts, esters and chelates of any mineral compound.

The composition of the invention also may contain emulsifiers and stabilizers such as soy lecithin, carrageenan, and combinations thereof. The composition of the invention may optionally contain other substances that may have a beneficial effect such as lactoferrin, nucleotides, nucleosides, immunoglobulins, and combinations thereof.

In one embodiment of the invention, *B. longum* AH1206 may be administered in combination with one or more probiotics. The term "probiotic" means a micro-organism that exerts beneficial effects on the health of the host. Any probiotic known in the art may be acceptable in this embodiment provided it achieves the intended result. In a particular embodiment, the probiotic may be selected from *Lactobacillus rhamnosus* GG (LGG), *Bifidobacterium longum species,* and *Bifidobacterium animalis subsp. lactis* BB-12. In an embodiment, the additional probiotic(s) may be viable or non-viable.

In another embodiment of the invention, *B. longum* AH1206 may be combined with one or more prebiotics. The term "prebiotic" means a non-digestible food ingredient that stimulates the growth and/or activity of probiotics. Any prebiotic known in the art will be acceptable in this embodiment provided it achieves the desired result. Prebiotics useful in the present invention may include oligosaccharides, polysaccharides, and other prebiotics that contain fructose, xylose, soya, galactose, glucose and mannose. More specifically, prebiotics useful in the present invention may include lactulose, gluco-oligosaccharide, inulin, polydextrose, galacto-oligosaccharide, fructo-oligosaccharide, isomalto-oligosaccharide, soybean oligosaccharides, lactosucrose, xylo-oligosacchairde, and gentio-oligosaccharides.

In yet another embodiment of the present invention, the formulation may contain other active agents such as long chain polyunsaturated fatty acids (LCPUFAs). Suitable LCPUFAs include, but are not limited to, α-linoleic acid, Y-linoleic acid, linoleic acid, linolenic acid, eicosapentanoic acid (EPA), arachidonic acid (ARA) and/or docosahexaenoic acid (DHA). In an embodiment, *B. longum* AH1206 is administered in combination with DHA. In another embodiment, *B. longum* AH1206 is administered in combination with ARA. In yet another embodiment, *B. longum* AH1206 is administered in combination with both DHA and ARA. Commercially available infant formula that contains DHA, ARA, or a combination thereof may be supplemented with *B. longum* AH1206 and used in the present invention. For example, Enfamil® LIPIL®, which contains effective levels of DHA and ARA, is commercially available and may be supplemented with *B. longum* AH1206 and utilized in the present invention.

In one embodiment, both DHA and ARA are administered in combination with *B. longum* AH1206. In this embodiment, the weight ratio of ARA:DHA may be from about 1:3 to about 9:1. In one embodiment of the present invention, this ratio is from about 1:2 to about 4:1. In yet another embodiment, the ratio is from about 2:3 to about 2:1. In one particular embodiment the ratio is about 2:1. In another particular embodiment of the invention, the ratio is about 1:1.5. In other embodiments, the ratio is about 1:1.3. In still other embodiments, the ratio is about 1:1.9. In a particular embodiment, the ratio is about 1.5:1. In a further embodiment, the ratio is about 1.47:1.

In certain embodiments of the invention, the level of DHA is in the range of about 0.0% and 1.00% of fatty acids, by weight. The level of DHA may be about 0.32% by weight. In some embodiments, the level of DHA may be about 0.33% by weight. In another embodiment, the level of DHA may be about 0.64% by weight. In another embodiment, the level of DHA may be about 0.67% by weight. In yet another embodiment, the level of DHA may be about 0.96% by weight. In a further embodiment, the level of DHA may be about 1.00% by weight.

In embodiments of the invention, the level of ARA is in the range of 0.0% and 0.67% of fatty acids, by weight. In another embodiment, the level of ARA may be about 0.67% by weight. In another embodiment, the level of ARA may be about 0.5% by weight. In yet another embodiment, the level of DHA may be in the range of about 0.47% and 0.48% by weight.

If included, the effective amount of DHA in an embodiment of the present invention is typically from about 3 mg per kg of body weight per day to about 150 mg per kg of body weight per day. In one embodiment of the invention, the amount is from about 6 mg per kg of body weight per day to about 100 mg per kg of body weight per day. In another embodiment the amount is from about 10 mg per kg of body weight per day to about 60 mg per kg of body weight per day. In yet another embodiment the amount is from about 15 mg per kg of body weight per day to about 30 mg per kg of body weight per day.

If included, the effective amount of ARA in an embodiment of the present invention is typically from about 5 mg per kg of body weight per day to about 150 mg per kg of body weight per day. In one embodiment of this invention, the amount varies from about 10 mg per kg of body weight per day to about 120 mg per kg of body weight per day. In another embodiment, the amount varies from about 15 mg per kg of body weight per day to about 90 mg per kg of body weight per day. In yet another embodiment, the amount varies from about 20 mg per kg of body weight per day to about 60 mg per kg of body weight per day.

If an infant formula is utilized, the amount of DHA in the infant formula may vary from about 5 mg/100 kcal to about 80 mg/100 kcal. In one embodiment of the present invention, DHA varies from about 10 mg/100 kcal to about 50 mg/100 kcal; and in another embodiment, from about 15 mg/100 kcal to about 20 mg/100 kcal. In a particular embodiment of the present invention, the amount of DHA is about 17 mg/100 kcal.

If an infant formula is utilized, the amount of ARA in the infant formula may vary from about 10 mg/100 kcal to about 100 mg/100 kcal. In one embodiment of the present invention, the amount of ARA varies from about 15 mg/100 kcal to about 70 mg/100 kcal. In another embodiment, the amount of ARA varies from about 20 mg/100 kcal to about 40 mg/100 kcal. In a particular embodiment of the present invention, the amount of ARA is about 34 mg/100 kcal.

If an infant formula is used, the infant formula may be supplemented with oils containing DHA and ARA using standard techniques known in the art. For example, DHA and ARA may be added to the formula by replacing an equivalent amount of an oil, such as high oleic sunflower oil, normally present in the formula. As another example, the oils containing DHA and ARA may be added to the formula by replacing an equivalent amount of the rest of the overall fat blend normally present in the formula without DHA and ARA.

If utilized, the source of DHA ,and ARA may be any source known in the art such as marine oil, fish oil, single cell oil, egg yolk lipid, and brain lipid. In some embodiments, the DHA and ARA are sourced from the single cell Martek oil, DHASCO®, or variations thereof. The DHA and ARA can be in natural form, provided that the remainder of the LCPUFA source does not result in any substantial deleterious effect on the infant. Alternatively, the DHA and ARA can be used in refined form.

In an embodiment of the present invention, sources of DHA and ARA are single cell oils as taught in U.S. Pat. Nos. 5,374,567; 5,550,156; and 5,397,591, the disclosures of which are incorporated herein in their entirety by reference. However, the present invention is not limited to only such oils.

In one embodiment, a LCPUFA source which contains EPA is used in combination with *B. longum* AH1206. In another embodiment, a LCPUFA source which is substantially free of EPA is used in combination with *B. longum* AH1206. For example, in one embodiment of the present invention, an infant formula containing less than about 16 mg EPA/100 kcal is supplemented with *B. longum* AH1206 and used in the method of the present invention. In another embodiment, an infant formula containing less than about 10 mg EPA/100 kcal is supplemented with *B. longum* AH1206 and used in the method of the present invention. In yet another embodiment, an infant formula containing less than about 5 mg EPA/100 kcal is supplemented with B. *longum* AH1206 and used in the method of the present invention. Another embodiment of the invention includes an infant formula supplemented with *B. longum* AH1206 that is free of even trace amounts of EPA.

It will be appreciated that the combination of *B. longum* AH1206 and probiotics, prebiotics, LCPUFAs or other active ingredients may be administered in a single formulation or as separate formulations administered at the same or different times and using the same or different routes of administration.

In one embodiment of the invention, the formulation may be used in immunization and vaccination protocols. Oral immunization using probiotic bacteria as vectors would not only protect the host from infection, but may replace the immunological stimuli that the pathogen would normally elicit, thus contributing to the immunological education of the host.

In an embodiment of the invention, the formulation is useful in treating, reducing, and/or preventing inflammation. As used herein, the term "treating" means ameliorating, improving or remedying a disease, disorder, or symptom of a disease or condition. The term "reducing" means to diminish in extent, amount, or degree. The term "preventing" means to stop or hinder a disease, disorder, or symptom of a disease or condition through some action.

In a particular embodiment, the invention comprises the use of *B. longum* AH1206 for the manufacture of a composition for reducing or preventing inflammation in an infant or child. Examples 2-6 each indicate that administration of *B. longum* AH1206 may reduce or prevent inflammation in an infant or child. The inflammation may be reduced or prevented in the gastrointestinal tract, the respiratory tract, or may be reduced systemically. The reduction or prevention of inflammation may improve allergies or asthma.

In this embodiment, the inflammation may be respiratory inflammation, such as asthma, allergic rhinitis, sinusitis, airway tissue inflammation, upper respiratory infection, influenza, croup, respiratory syncytial virus, bronchitis, bronchiolitis, pneumonia, or airway lumen inflammation. Similarly, the inflammation may be gastrointestinal inflammation, such as diarrhea, inflammatory bowel disease, Crohn's disease, enterocolitis, ulcerative colitis, allergic colitis, irritable bowel syndrome, pouchitis, post-infection colitis, *Clostridium difficile*-associated diarrhea, Rotavirus-associated diarrhea, or post-infective diarrhea, or diarrheal disease due to an infectious agent, such as *E. coli.* In other embodiments, the inflammation may be systemic, such as in rheumatoid arthritis. The term "systemic", as used herein, means relating to or affecting the entire body.

In some embodiments, the reduction or prevention of inflammation may ameliorate or prevent atopic conditions or diseases. For example, the reduction or prevention of inflammation may ameliorate or prevent atopic dermatitis. In a particular embodiment, the reduction or prevention of inflammation may ameliorate or prevent common infant or childhood illnesses. For example, the reduction or prevention of inflammation may ameliorate or prevent diarrhea or acute otitis media. In yet another embodiment, the reduction or prevention of inflammation may ameliorate or prevent food allergies.

It is believed that *B. longum* AH1206 acts by antagonizing and excluding pro-inflammatory micro-organisms from the gastrointestinal tract or inflammatory site. It is also believed that *B. longum* AH1206 acts by reducing the levels of pro-inflammatory cytokines.

In a particular embodiment, the strain may modify the levels of IL-10 in a subject. IL-10 is produced by T cells, B cells, monocytes and macrophages. This cytokine augments the proliferation and differentiation of B cells into antibody secreting cells. IL-10 exhibits mostly anti-inflammatory activities. It up-regulates IL-1 RA expression by monocytes and suppresses the majority of monocyte inflammatory activities. IL-10 inhibits monocyte production of cytokines, reactive oxygen and nitrogen intermediates, major histocompatibility complex (MHC) class II expression, parasite killing and IL-10 production via a feed back mechanism. This cytokine has also been shown to block monocyte production of intestinal collagenase and type IV collagenase by interfering with a prostaglandin E2-cyclic adenosine monophosphate (PGE₂-cAMP) dependant pathway and therefore may be an important regulator of the connective tissue destruction seen in chronic inflammatory diseases. Thus, in an embodiment, the strain may upregulate, or induce secretion of, IL-10 levels in infants or children consuming the strain.

In other embodiments, *B. longum* AH1206 downregulates or decreases secretion of TNF-α, IL-2, IL-4, IL-5, and/or IFN-γ or other pro-inflammatory cytokines or chemokines. TNF-α, a pro-inflammatory cytokine, initiates a cascade of cytokines and biological effects resulting in an inflammatory state. In some embodiments, the percentage of T regulatory cells may be increased upon *B. longum* AH1206 administration. In other embodiments, the administration of *B. longum* AH1206 inhibits the induction of an OVA-specific IgE response.

In some embodiments, the formulation of the invention is useful for maintaining the homeostasis of the immune system. In other embodiments, the formulation of the invention is useful for improving or enhancing immunity in a subject.

In some embodiments, the enhancement of immunity or suppression of inflammation may include stimulation of intestinal integrity; reduction of intestinal permeability; improvement of mucin synthesis, secretion, and/or quality; improvement of the maturation and differentiation of the intestinal epithelium; improvement of nutrient absorption; increase of the production of soluble factors that transfer antimicrobial activity; stimulation of, improvement of, or support of resistance to infection; support of cellular or humoral responses against viral or bacterial infection; increased cytotoxicity (both anti-viral and anti-tumor); support of systemic and/or mucosal vaccination responses; increase or support of cellular and/or humoral immunity; increase or support of natural immunity (including neutrophils, phagocytes, macrophages, and natural killer cell activity); increase or support of adaptive T and B cell immunity; stimulation of a helper T cell 1 (Th1) cytokine pattern (increased IL-1, IL-2, IFN-Y, IL-12, TNF-α; human leukocyte antigen-Dr (HLA-Dr) expression); suppression of inflammation or production of systemic and mucosal inflammatory mediators (including cytokines and/or chemokines); reduction of sensitization by reducing total and/or allergen-specific IgE; reduction of the production of allergic cytokines; reduction of a Th2 supporting immunoglobulin profile; and combinations thereof.

The production of multifunctional cytokines across a wide spectrum of tumor types suggests that significant inflammatory responses are ongoing in patients with cancer. Thus, in an embodiment of the invention, *B. longum* AH1206 may be useful in treating the symptoms of cancer. Due to the anti-inflammatory properties of *B. longum* AH1206, this bacterial strain may reduce the rate of malignant cell transformation. Furthermore, intestinal bacteria can produce, from dietary compounds, substances with genotoxic, carcinogenic and tumor-promoting activity and gut bacteria can activate procarcinogens to DNA reactive agents. In general, species of *Bifidobacterium* have low activities of xenobiotic metabolizing enzymes compared to other populations within the gut such as bacteroides, eubacteria and clostridia. Therefore, increasing the number of *Bifidobacterium* bacteria in the gut could beneficially modify the levels of these enzymes.

In particular embodiments, *B. longum* AH1206 may be administered in combination with anti-inflammatory therapies such as non-steroid anti-inflammatory drugs (NSAIDs) or infliximab.

The infant gut microflora is rapidly established in the first few weeks following birth. The nature of this intestinal colonization is initially determined by early exposure to environmental sources of microbes as well as the health of the infant. Infants born via cesarean section, preterm or other infants who spend the first portion of their lives in a sterile incubator, and/or infants that are administered antibiotics early in life are likely to have significant delays in the development of a healthy gut microflora. Because these infants do not have the opportunity to acquire a healthy gut microflora from their mothers or environment, consumption of *B. longum* AH1206 may beneficially contribute to the proper development and function of the intestinal immune system.

In some embodiments of the present invention, the infant or child is in need of the treatment, reduction, or prevention of inflammation. The subject may be at risk for inflammation due to genetic predisposition, diet, lifestyle, diseases, or disorders. For example, a preterm or immunosuppressed infant may be at risk for inflammation and may, therefore, be in need of such treatment, reduction, or prevention.

The composition of the invention can be packaged in any type of container known in the art to be used for storing nutritional products such as glass, lined paperboard, plastic, and coated metal cans. In some embodiments, the composition is packaged via blow-fill-seal packaging techniques. In other embodiments, the composition is provided in a single dose container. The packaging of the composition may be conducted under aseptic conditions. In some embodiments, the composition is prepared such that it is acceptable for direct delivery to a preterm infant via nasogastric tubes, nasoduodenal tubes, or nasojejunal tubes.

The composition of the invention may be shelf stable. By "shelf stable," it is meant that the composition, in a form that is ready to consume, remains in a single homogenous phase (i.e., does not separate into more than one phase upon visual inspection), and/or that settling does not occur upon visual inspection after storage overnight in the refrigerator.

The following examples describe various embodiments of the present invention. Other embodiments within the scope of the claims herein will be apparent to one skilled in the art from consideration of the specification or practice of the invention as disclosed herein. It is intended that the specification, together with the examples, be considered to be exemplary only, with the scope and spirit of the invention being indicated by the claims which follow the examples. In the examples, all percentages are given on a weight basis unless otherwise indicated.

### Example 1

This example illustrates the characterization of bacteria isolated from infant feces.

### Isolation of Probiotic Bacteria

Fresh feces were obtained from a 2-day-old male breast-fed infant and serial dilutions were plated on TPY (trypticase, peptone and yeast extract) and MRS (deMann, Rogosa and Sharpe) media supplemented with 0.05% cysteine and mupirocin. Plates were incubated in anaerobic jars (BBL, Oxoid) using CO₂ generating kits (Anaerocult A, Merck) for 2-5 days at 37°C. Gram positive, catalase negative rod-shaped or bifurcated/pleomorphic bacteria isolates were streaked for purity on to complex non-selective media (MRS and TPY). Isolates were routinely cultivated in MRS or TPY medium unless otherwise stated at 37°C under anaerobic conditions. Presumptive *Bifidobacterium* were stocked in 40% glycerol and stored at -20°C and -80°C.

Following isolation of a pure bifidobacteria strain, assigned the designation AH1206, microbiological characteristics were assessed and are summarized in Table 1 below. AH1206 is a gram positive, catalase negative pleomorphic shaped bacterium which is Fructose-6-Phoshate Phosphoketolase positive confirming its identity as a bifidobacterium. Using minimal media in which a single carbon source was inserted, AH1206 was able to grow on all carbon sources tested (Glucose, Lactose, Ribose, Arabinose, Galactose, Raffinose, Fructose, Malt Extract, Mannose, Maltose, Sucrose).

**Table 1. Physiochemical characteristics of B. longum AH1206**

| **Strain Characteristics** | ***B. longum* AH1206** |
|---|---|
| Gram Stain | + |
| Catalase | - |
| Motil ity | - |
| F6PPK* | + |
| Milk coagulation | + |
| 45°C anaerobic culture | - |
| 45°C aerobic culture | - |

| **Carbohydrate Fermentation** | |
|---|---|
| *Glucose* | + |
| *Lactose* | + |
| *Ribose* | + |
| *Arabinose* | + |
| *Galactose* | + |
| *Raffinose* | + |
| *Fructose* | + |
| *Malt Extract* | + |
| *Mannose* | + |
| *Maltose* | + |
| *Sucrose* | + |

| | |
|---|---|
| * signifies Fructose-6-Phoshate Phosphoketolase Assay | |

### Species identification

16s Intergenic spacer (IGS) sequencing was performed to identify the species of bifidobacteria isolated. Briefly, DNA was isolated from AH1206 using 100 µl of Extraction Solution and 25 µl of Tissue Preparation solution (Sigma, XNAT2 Kit). The samples were incubated for 5 minutes at 95°C and then 100 µl of Neutralization Solution (XNAT2 kit) was added. Genomic DNA solution was quantified using a Nanodrop spectrophotometer and stored at 4°C. PCR was performed using the IGS primers, IGS L: 5'-GCTGGATCACCTCCTTTC-3' (SEQ ID No. 3) which is based on SEQ ID NO. 1 and IGS R: 5'-CTGGTGCCAAGGCATCCA-3' (SEQ ID No. 4) which is based on SEQ ID NO. 2. The cycling conditions were 94°C for 3 min (1 cycle), 94°C for 30 sec, 53°C for 30 sec, 72°C for 30 sec (28 cycles). The PCR reaction contained 4 µl (50ng) of DNA, PCR mix (XNAT2 kit), 0.4 µM IGS L and R primer (MWG Biotech, Germany). The PCR reactions were performed on an Eppendorf thermocycler. The PCR products (10 µl) were run alongside a molecular weight marker (100 bp Ladder, Roche) on a 2 % agarose EtBr stained gel in TAE, to determine the IGS profile. PCR products of Bifidobacterium (single band) were purified using the Promega Wizard PCR purification kit. The purified PCR products were sequenced using the primer sequences (above) for the intergenic spacer region. Sequence data was then searched against the National Center for Biotechnology Information (NCBI) nucleotide database to determine the identity of the strain by nucleotide homology. The resultant DNA sequence data was subjected to the NCBI standard nucleotide-to-nucleotide homology BLAST search engine (http://www.ncbi.nlm.nih.gov/BLAST/). The nearest match to the sequence was identified and then the sequences were aligned for comparison using DNASTAR MegAlign software. The sequences obtained can be viewed in the sequence listing in which SEQ ID NO. 1 is the IGS forward sequence and SEQ ID NO. 2 is the IGS reverse sequence. Searching the NCIMB database revealed that AH1206 has a unique IGS sequence with its closest sequence homology to a *Bifidobacterium longum.* A paper copy and computer readable format of the Sequence Listing for Table 3 have been submitted herewith and are hereby incorporated by reference in their entirety. The computer readable file on the disc is identified as AH1206_ST25.txt, Size: 2 KB, Created September 12, 2008.

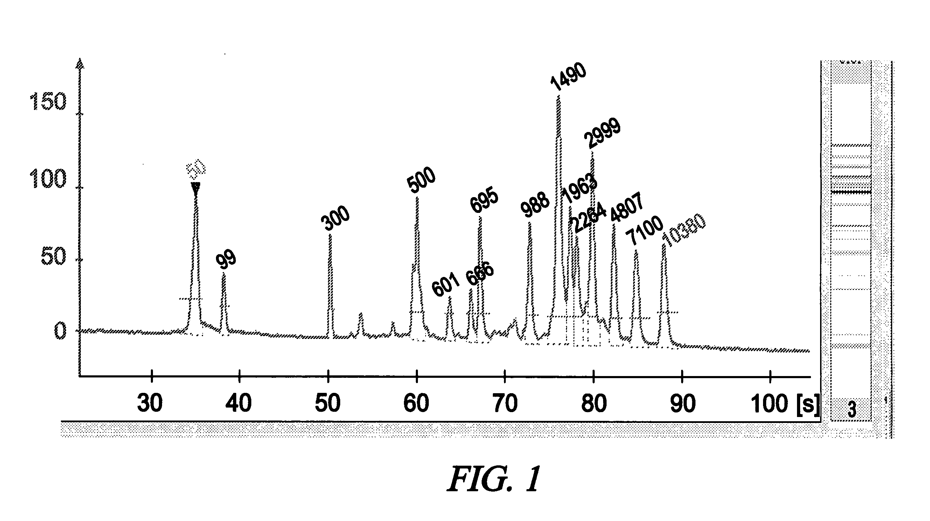

In order to develop a barcode PCR profile for AH1206, PCR was performed using BOX primers. The cycling conditions were 94°C for 7 min (1 cycle); 94°C for 1 minute, 65°C for 8 minutes, (30 cycles) and 65°C for 16 minutes. The PCR reaction contained 50 ng of DNA, PCR mix (XNAT2 kit) and 0.3 µM BOXA1 R primer (5'-CTACGGCAAGGCGACGCTGACG-3') (SEQ ID No. 5) (MWG Biotech, Germany). The PCR reactions were performed on an Eppendorf thermocycler. The PCR products (1 µl) were run alongside a molecular weight marker (DNA 7500 ladder, Agilent, Germany) using the DNA 7500 LabChip® on the Agilent 2100 Bioanalyzer (Agilent, Germany). The barcode (PCR product profile) was determined using the Agilent Bioanalyzer software where peak number (PCR products) and size were identified (Fig. 1).

### Antibiotic sensitivity profiles

Antibiotic sensitivity profiles of the *B. longum* strain were determined using the "disc susceptibility" assay. Cultures were grown up in the appropriate broth medium for 24-48 hours, spread-plated (100µl) onto agar media, and discs containing known concentrations of the antibiotics were placed onto the agar. Strains were examined for antibiotic sensitivity after 1-2 days incubation at 37°C under anaerobic conditions. Strains were considered sensitive if zones of inhibition of 1 mm or greater were seen. The minimum inhibitory concentration (MIC) for each antibiotic was independently assessed. The MIC for clindamycin, vancomycin and metronidazole were 0.32, 0.75 and 0.38 respectively.

### Intestinal transit

To determine whether *Bifidobacterium longum* could survive at low pH values equivalent to those found in the stomach, bacterial cells were harvested from fresh overnight cultures, washed twice in phosphate buffer (pH 6.5) and resuspended in TPY broth adjusted to pH 2.5 (with 1 M HCl). Cells were incubated at 37°C and survival was measured at intervals of 5, 30, 60 and 120 minutes using the plate count method. AH1206 survived well for 5 minutes at pH 2.5 while no viable cells were recovered after 30 minutes.

Upon exiting the stomach, putative probiotics are exposed to bile salts in the small intestine. In order to determine the ability of *B. longum* to survive exposure to bile, cultures were streaked on TPY agar plates supplemented with 0.3% (w/v), 0.5%, 1 %, 2%, 5%, 7.5% or 10% porcine bile. *B. longum* AH1206 growth was observed on plates containing up to 1% bile.

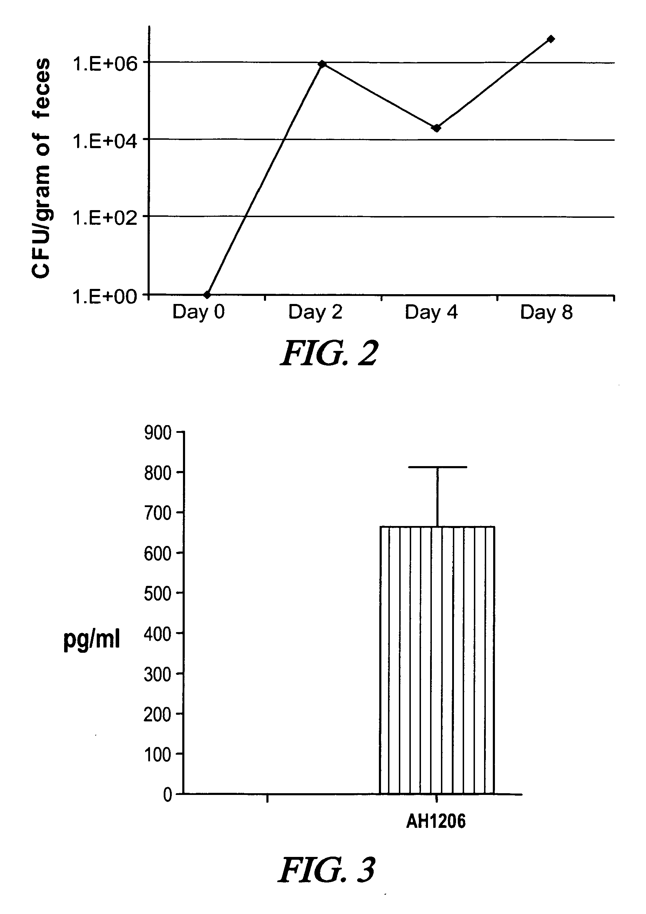

In a murine model, the ability of *B. longum* AH1206 to transit the gastrointestinal tract was assessed. Mice consumed 1x10⁹ AH1206 daily and fecal pellets were examined for the presence of the fed micro-organism. Detection of AH1206 was facilitated by isolating a spontaneous rifampicin-resistant variant of the bifidobacteria. Incorporation of rifampicin in the TPY plates was used to assess transit and ensured that only the fed rifampicin-resistant bifiobacteria was cultured. Fecal samples were collected daily and *B. longum* transit through the gastrointestinal tract was confirmed (Fig. 2).

### Anti-microbial activity

The indicator pathogenic micro-organisms used in this study were propagated in the following medium under the following growth conditions: *Salmonella typhimurium* (37°C, aerobic) in Tryptone Soya broth/agar supplemented with 0.6% yeast extract (TSAYE, Oxoid), *Campylobacter jejuni* (37°C, anaerobic) and *E. coli* 0157: H7 (37°C, anaerobic) on Blood agar medium, and *Clostridium difficile* (37°C, anaerobic) in reinforced Clostridial medium (RCM, Oxoid). All strains were inoculated into fresh growth medium and grown overnight before being used in experiments.

Antimicrobial activity was detected using the deferred method. Briefly, *B. longum* AH1206 was incubated for 36-48 hours. Ten-fold serial dilutions were spread-plated (100µl) onto TPY agar medium. After overnight incubation, plates with distinct colonies were overlayed with the indicator bacterium. The indicator lawn was prepared by inoculating a molten overlay with 2% (v/v) of an overnight indicator culture which was poured over the surface of the inoculated TPY plates. The plates were re-incubated overnight under conditions suitable for growth of the indicator bacterium. Indicator cultures with inhibition zones greater than 1 mm in radius were considered sensitive to the test bacterium. *B. longum* AH1206 inhibited the growth of all pathogenic organisms tested, with zones of clearing measuring 14, >80, 13.33 and 17 mm for *Salmonella typhimurium, Campylobacter jejuni, E.* coli O157: H7, and *Clostridium difficile* respectively.

### Example 2

This example illustrates cytokine production by PBMCs in response to B. *longum.* PBMCs were isolated from healthy donors by density gradient centrifugation. PBMCs were stimulated with the probiotic bacterial strain for a 72 hour period at 37°C. At this time culture supernatants were collected, centrifuged, aliquoted, and stored at - 70°C until being assessed for IL-10 levels using cytometric bead arrays (BD BioSciences). AH1206 induced significant secretion of the anti-inflammatory cytokine IL-10 by human PBMCs, suggesting this strain may be useful as an anti-inflammatory agent *in vivo* (Fig. 3). As an anti-inflammatory agent, it may be useful in reducing and preventing inflammation in humans.

### Example 3

This example illustrates the effect of *B. longum* AH1206 on the attenuation of respiratory disease in a murine model of asthma. This study utilized a Balb/c OVA-sensitized mouse model of allergic airway inflammation. Mice were sensitized by intraperitoneal injection of OVA and disease was initiated by intranasal challenge with OVA. Twenty-four hours after the last challenge (day 15), mice were subjected to measurements of airway responsiveness followed by BAL procedure. OVA-sensitized, saline-challenged mice served as controls. Commencing on day 1 (i.e. at time of first OVA sensitization), animals received *B. longum* AH1206 via a gavaging needle for 14 consecutive days. Animals gavaged with MRS broth served as controls.

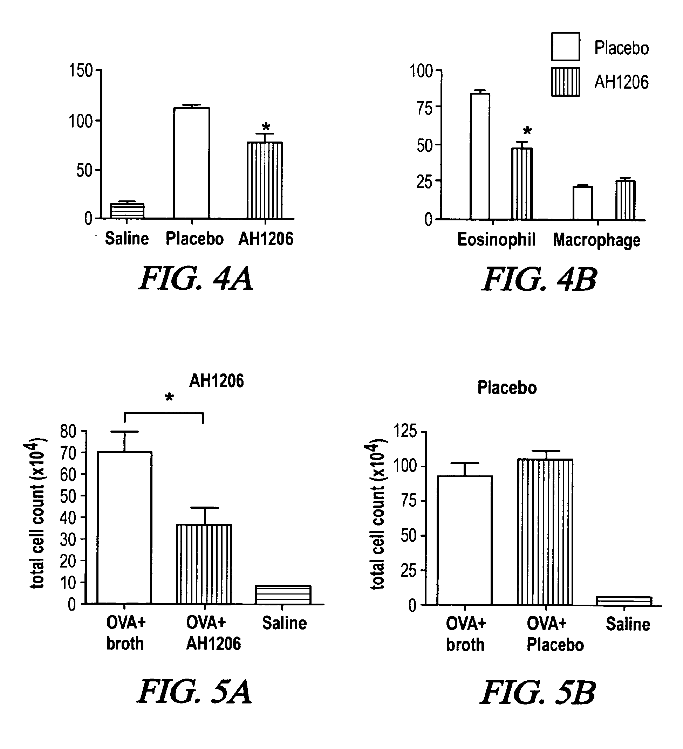

Airway inflammation was assessed by inflammatory cell counts in BAL fluid. Cells were removed from BAL fluid by centrifugation and cells were resuspended in phosphate-buffered saline (1 ml). BAL cells were stained with trypan blue, and viable cells were counted using a hemocytometer. Smears of BAL cells were prepared with a Cytospin (Thermo Shandon, Pittsburgh, PA) and stained with HEMA 3 reagent (Biochemical Sciences, Swedesboro, NJ) for differential cell counts, where a total of 200 cells were counted for each lavage. Consumption of *B. longum* AH1206 significantly reduced the total BAL counts compared to placebo with the majority of this difference being seen in the eosinophil population (Figure 4).

This study was repeated to further investigate whether the probiotic bacteria strain *Bifidobacterium longum* AH1206 suppresses allergic responses in an OVA-sensitized mouse model of allergic airway inflammation. Briefly, adult male BALB/c mice were sensitized by intraperitoneal injection of OVA on day 0 and day 6. On days 12 and 14, mice were challenged intranasally with OVA. Twenty-four hours after the last challenge (day 15), mice were subjected to measurements of airway responsiveness followed by BAL procedure. OVA/alum-sensitized, saline-challenged mice served as controls. Animals received probiotic or placebo throughout the trial. Airway inflammation (cytokine and cell counts) was assessed by inflammatory cell counts in BAL fluid. Airway responsiveness was also measured using the Buxco whole-body plethysmograph. Splenocytes were also isolated from OVA-sensitized mice and were incubated in the presence of anti-CD3 and anti-CD28 antibodies after which cytokine levels were measured in the supernatants by flow cytometry.

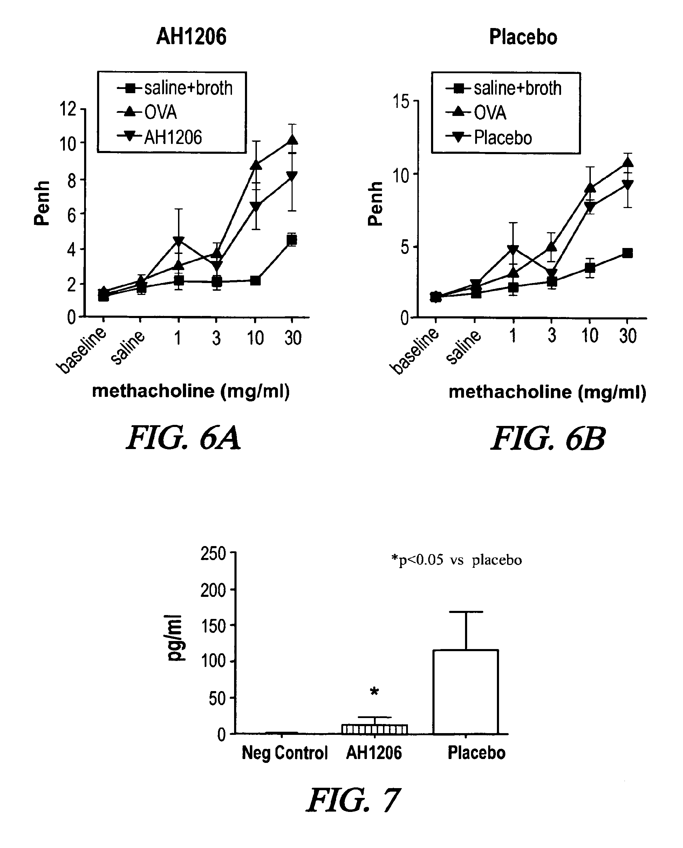

*B. longum* AH1206 treatment resulted in a significant reduction in cells recovered from BAL fluid following OVA challenge, when compared to broth fed animals (Fig. 5). Airway responsiveness was measured and the OVA-sensitized mice showed an enhancement of airway hyperresponsiveness (AHR) to methacholine when compared with saline-challenged mice. However no modulation of this enhanced airway responsiveness to methacholine, as assessed by changes in enhanced pause was seen (Fig. 6).

BAL cytokine levels were measured by cytometric bead array no significant differences were noted for IL-10, IFN-Y, IL-6 and CCL2 levels. AH1206 significantly reduced TNF-α levels compared to OVA control (Fig. 7).

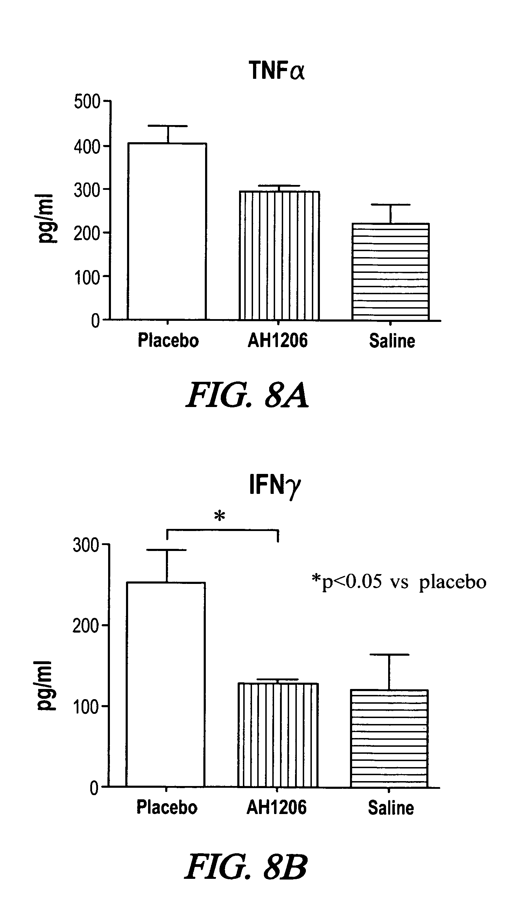

Cytokine levels in splenocyte supernatants were quantified by cytometric bead array (CBA) following in vitro OVA or anti-CD3 anti-CD28 stimulation. Increased IL-10 release from OVA stimulated splenocytes, associated with in vivo OVA sensitization, was not observed in AH1206-fed mice. There was no significant difference in IL-6, TNF and MCP-1 (CCL2) levels. IL-10 release from CD3/CD28 splenocytes was not increased in AH1206-fed animals. However, secretion of the pro-inflammatory cytokines TNF-α and IFN-Y was significantly reduced in the splenocyte culture supernatants of AH1206-fed animals (Fig. 8). No significant changes were noted for the other cytokines measured.

### Example 4

The aim of this study was to investigate whether the probiotic bacteria, *Bifidobacterium longum* AH1206 suppresses allergic responses in an OVA-induced allergy mouse model. BALB/c mice were divided into groups (8/group) and fed placebo, *Bifidobacterium longum* AH1206, or distilled water for four weeks. All mice were orally gavaged weekly with Ovalbumin and Cholera Toxin in 300µls of phosphate buffered saline (PBS), excluding one of the distilled water groups which were orally gavaged with 300µls PBS only as a control. After four weeks of treatment, a blood sample from each mouse was collected via facial vein puncture and a subsequent enzyme-linked immunosorbent assay (ELISA) performed to measure OVA-specific IgE levels. The spleens and mesenteric lymph node cells were isolated and stimulated *in vitro* with LPS and antiCD3/CD28 and the immunodominant OVA peptide. Th1 and Th2 cytokines were measured by cytometric CBA.

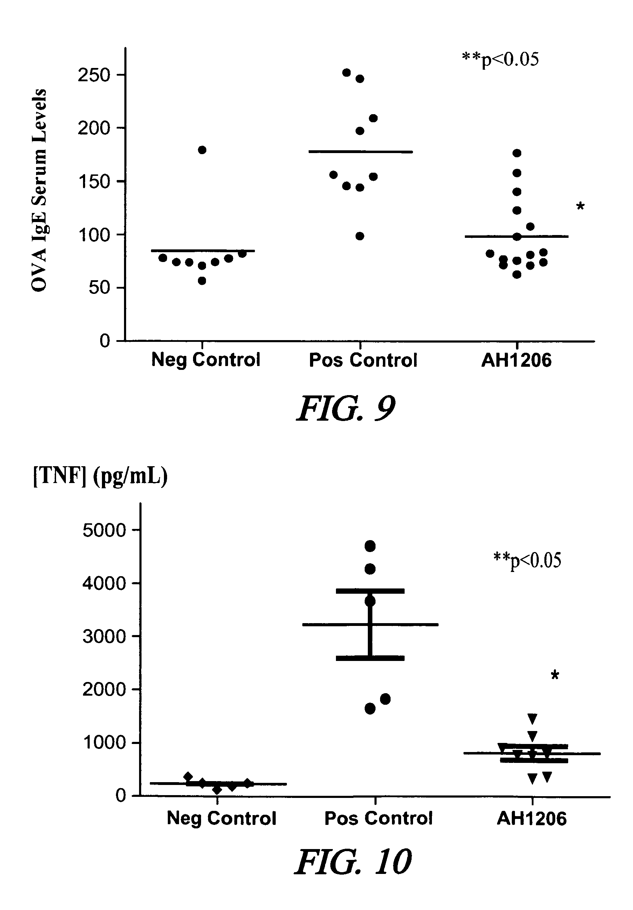

There was significantly less OVA-specific IgE induced in the AH1206-fed group compared to the placebo and positive control groups (Fig. 9). The negative control group and the AH1206-fed groups were not different, suggesting that AH1206-feeding completely prevented the induction of an OVA-specific IgE response. This example further suggests that AH1206 administration can prevent the onset of inflammation in humans. Statistics were calculated using the unpaired T test.

Splenocytes were isolated from probiotic, placebo, and distilled water-fed BALB/c mice and either left unstimulated or stimulated with lipopolysaccharide (LPS), antiCD3/CD28, or the immunodominant OVA peptide and then analyzed for cytokine production of TNF-α, IL-2, IFN-Y, IL-4 and IL-5 by Th1/Th2 cytometric bead array. Cytokine results are summarized in Table 2.

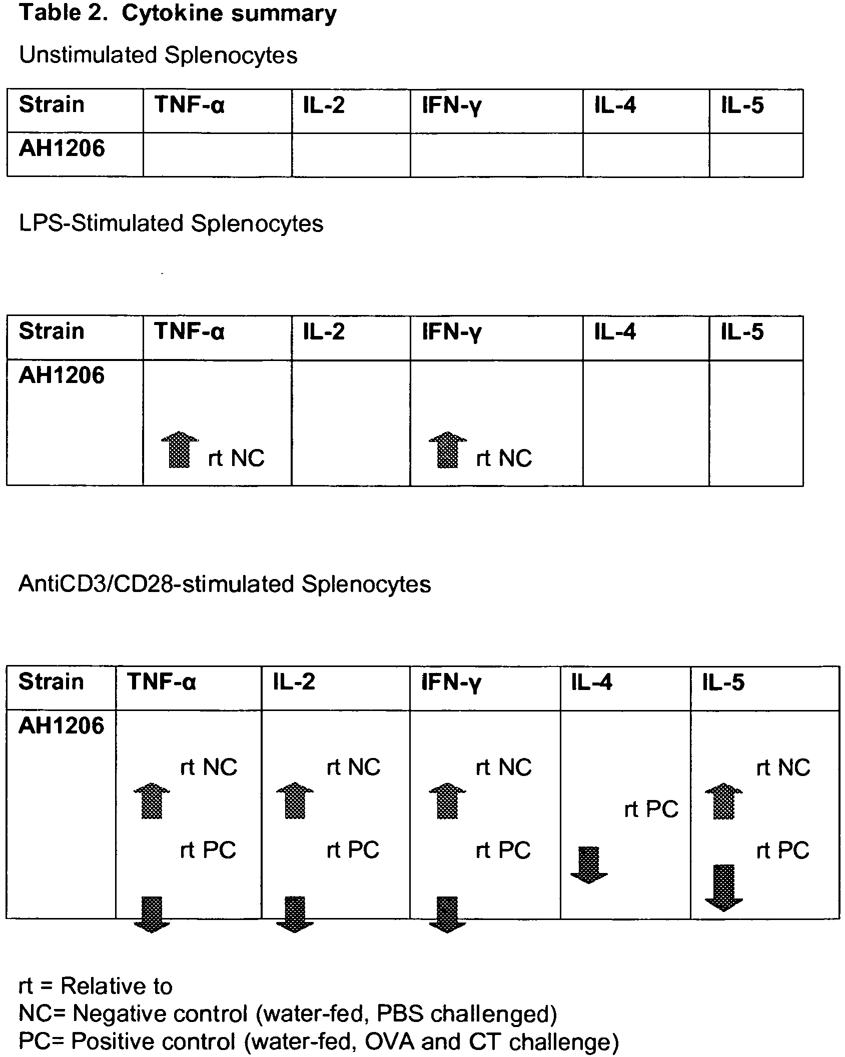

In unstimulated splenocytes, no alterations were observed compared to control animals. TNF-α and IFN-Y release from LPS-stimulated splenocytes was significantly greater for AH1206-fed animals compared to the negative controls but these levels were consistent with those observed with the OVA-sensitized and cholera toxin-administered positive controls. CD3/CD28 stimulation revealed profound alterations in lymphocyte signaling in the probiotic-fed group. AH1206-fed animals secreted significantly less TNF-α compared to the positive controls but levels were higher compared to negative controls (Fig. 10). AH1206-fed animals had significantly lower levels of IFN-Y, IL-2, IL-4 and IL-5 compared to the non-probiotic fed positive controls.

### Example 5

This study investigated the effect of probiotic consumption on regulatory T cell number and activity in healthy mice. BALB/c mice (10/group) were fed *Bifidobacterium longum* AH1206 or placebo for three weeks. Following probiotic/placebo consumption, CD4⁺CD25⁺ T regulatory cells were isolated and their *in vitro* suppressive activity was determined by measuring proliferation of anti-CD3/CD28 stimulated CFSE-labeled CD4⁺ responder T cells using flow cytometry. CD4⁺ responder T cells were co-incubated with CD4⁺CD25⁻T cells as a control. The percentage of CD4⁺CD25⁺ cells (regulatory T cells) in murine splenocytes that were also FoxP3 positive was determined in the spleens of probiotic or placebo-fed mice.

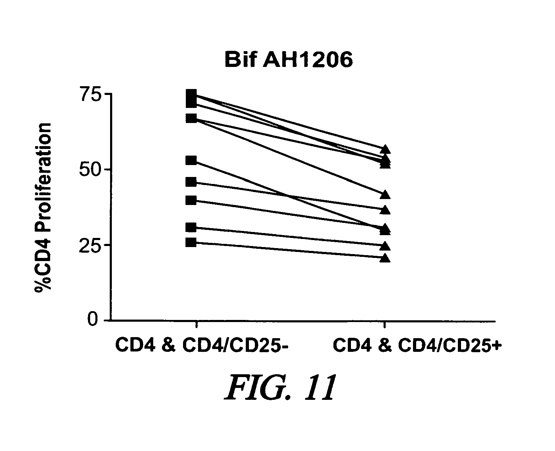

The percentage of CD4⁺ cells that proliferated when co-incubated with CD4⁺CD25⁺ cells from the probiotic/placebo fed mice was compared to the percentage of CD4⁺ cells that proliferated when co-incubated with CD4⁺CD25⁻ cells from the same trial mouse. In each case, T cell proliferation was less in cultures containing CD4⁺CD25⁺ cells compared to cultures containing CD4 cells alone and depleted of the CD25⁺ cells (Fig. 11).

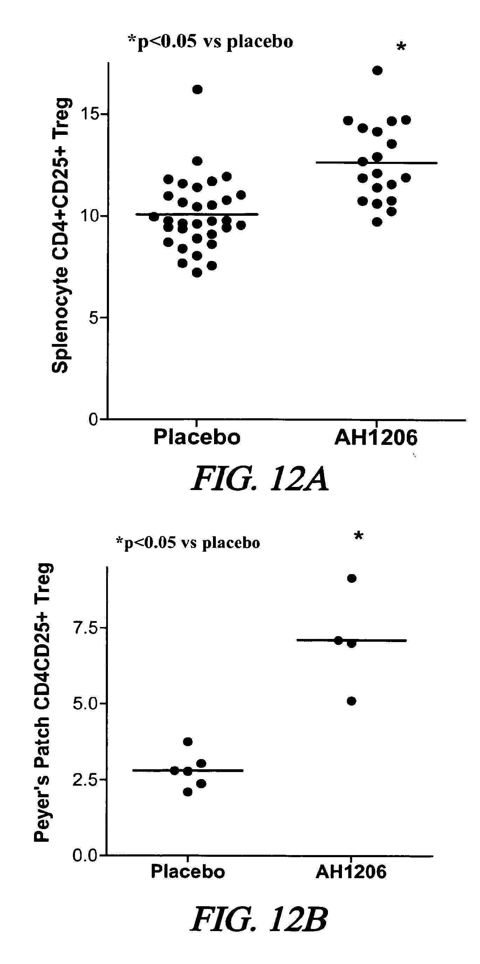

The percentage of cells in the CD4⁺ population that were also CD25⁺ was determined (Fig. 12). The *Bifidobacterium longum* AH1206-fed group had significantly more CD4⁺ T cells that were CD25⁺ (i.e. T regulatory cells) than their placebo-fed counterparts (p= 0.0081). This suggests that the percentage of T regulatory cells within the CD4⁺ population was increased significantly by feeding with AH1206. This result indicates that AH1206 administration may reduce or prevent inflammation within a human.

The number of CD4⁺CD25⁺FoxP3⁺ cells in the whole splenocyte populations of probiotic- or placebo-fed mice was also determined. The number of CD4⁺CD25⁺ T regulatory cells expressing FoxP3 was unchanged in the spleens of probiotic-fed mice relative to placebo or unfed mice.

### Example 6

Germ free mice were purchased at 6 weeks of age and maintained in the germ-free unit at the biological services unit in UCC. Animals consumed the probiotic strain *Bifidobacterium longum* AH1206 for 14 days or remained germ-free. Induction of T regulatory cells was assessed by flow cytometry.

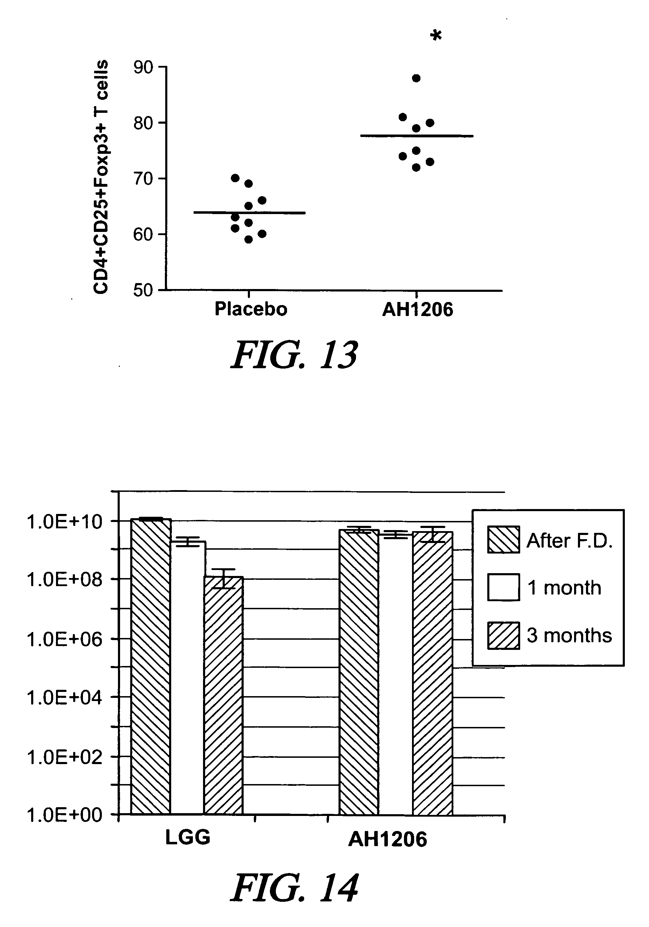

The numbers of CD4⁺CD25⁺Foxp3⁺ cells in the spleen of AH1206-fed germ-free animals was significantly increased following feeding with AH1206 (Fig. 13). Again, this result indicates that AH1206 administration may reduce or prevent inflammation within a human. Total CD3/CD4 or CD3/CD8 counts remained unaltered.

### Example 7: Stability results

The stability of probiotic strain-AH1206 was assessed over 3 months at 30°C (Fig. 13). The results indicate that LGG was a poor performer over the test period with a 2 log drop over the 3 month period whereas AH 1206 was quite stable with no viability loss recorded over the period.

All references cited in this specification, including without limitation, all papers, publications, patents, patent applications, presentations, texts, reports, manuscripts, brochures, books, internet postings, journal articles, and periodicals are hereby incorporated by reference into this specification in their entireties. The discussion of the references herein is intended merely to summarize the assertions made by their authors and no admission is made that any reference constitutes prior art. Applicants reserve the right to challenge the accuracy and pertinence of the cited references.

These and other modifications and variations to the present invention may be practiced by those of ordinary skill in the art, without departing from the spirit and scope of the present invention, which is more particularly set forth in the appended claims. In addition, it should be understood that aspects of the various embodiments may be interchanged both in whole or in part. Furthermore, those of ordinary skill in the art will appreciate that the foregoing description is by way of example only, and is not intended to limit the invention so further described in such appended claims.

## Claims

1. An infant formula comprising a protein source, a fat source, a carbohydrate source, and *B. longum* AH1206.

2. The infant formula of claim 1, wherein the infant formula comprises B. *longum* AH1206 in the range of about 1x10⁴ cfu to about 1x10¹⁰ cfu per gram infant formula.

3. The infant formula of claim 1, wherein the *B. longum* AH1206 is viable.

4. The infant formula of claim 1, wherein the *B. longum* AH1206 is non-viable.

5. The infant formula of claim 1, further comprising an additional probiotic.

6. The infant formula of claim 5, wherein the additional probiotic comprises LGG.

7. The infant formula of claim 1, further comprising a prebiotic.

8. The infant formula of claim 7, wherein the prebiotic is selected from the group consisting of lactulose, gluco-oligosaccharide, inulin, polydextrose, galacto-oligosaccharide, fructo-oligosaccharide, isomalto-oligosaccharide, soybean oligosaccharides, lactosucrose, xylo-oligosacchairde, and gentio-oligosaccharides.

9. The infant formula of claim 1, further comprising at least one LCPUFA.

10. The infant formula of claim 1, further comprising ARA and DHA.

11. A children's nutritional product comprising a protein source, a fat source, a carbohydrate source, and *B. longum* AH1206.

12. *B. longum* AH1206 for use in a method for reducing or preventing inflammation in an infant or child, the method comprising administering B. *longum* AH1206 to the infant or child.

13. *B. longum* AH1206 for use in the method of claim 12, wherein the reduction or prevention of inflammation improves allergies.

14. *B. longum* AH1206 for use in the method of claim 12, wherein the reduction or prevention of inflammation improves asthma.

15. *B. longum* AH1206 for use in the method of claim 12, wherein the reduction or prevention of inflammation improves an illness selected from the group consisting of diarrhea, respiratory infections, and otitis media.

## Patentansprüche

1. Säuglingsprodukt, umfassend eine Proteinquelle, eine Fettquelle, eine Kohlehydratquelle und *B. longum* AH1206.

2. Säuglingsprodukt nach Anspruch 1, wobei das Säuglingsprodukt B. *longum* AH1206 im Bereich von ungefähr 1x10⁴ cfu bis ungefähr 1x10¹⁰ cfu pro Gramm des Säuglingsprodukts umfasst.

3. Säuglingsprodukt nach Anspruch 1, wobei das *B. longum* AH1206 lebensfähig ist.

4. Säuglingsprodukt nach Anspruch 1, wobei das *B. longum* AH1206 nicht lebensfähig ist.

5. Säuglingsprodukt nach Anspruch 1, ferner umfassend ein zusätzliches Probiotikum.

6. Säuglingsprodukt nach Anspruch 5, wobei das zusätzliche Probiotikum LGG umfasst.

7. Säuglingsprodukt nach Anspruch 1, ferner umfassend ein Präbiotikum.

8. Säuglingsprodukt nach Anspruch 7, wobei das Präbiotikum ausgewählt ist aus der Gruppe bestehend aus Laktulose, Gluco-Oligosaccharid, Inulin, Polydextrose, Galacto-Oligosaccharid, Fructo-Oligosaccharid, Isomalto-Oligosaccharid, Sojabohnen-Oligosacchariden, Laktosukrose, Xylo-Oligosaccharid und Gentio-Oligosacchariden.

9. Säuglingsprodukt nach Anspruch 1, ferner umfassend mindestens eine LCPUFA.

10. Säuglingsprodukt nach Anspruch 1, ferner umfassend ARA und DHA.

11. Kindernahrungsprodukt, umfassend eine Proteinquelle, eine Fettquelle, eine Kohlehydratquelle und *B. longum* AH 1206.

12. *B. longum* AH1206 zur Verwendung in einem Verfahren zur Verminderung oder Verhütung einer Entzündung bei einem Säugling oder einem Kind, wobei das Verfahren die Verabreichung von *B. longum* AH1206 an den Säugling oder das Kind umfasst.

13. *B. longum* AH1206 zur Verwendung in dem Verfahren nach Anspruch 12, wobei die Verminderung oder Verhütung einer Entzündung Allergien verbessert.

14. *B. longum* AH1206 zur Verwendung in dem Verfahren nach Anspruch 12, wobei die Verminderung oder Verhütung einer Entzündung Asthma verbessert.

15. *B. longum* AH1206 zur Verwendung in dem Verfahren nach Anspruch 12, wobei die Verminderung oder Verhütung einer Entzündung eine Krankheit verbessert, ausgewählt aus der Gruppe bestehend aus Diarrhö, Atemwegsinfektionen und Otitis media.

## Revendications

1. Formule pour nourrisson comprenant une source de protéines, une source de lipides, une source de glucides, et *B. longum* AH1206*.*

2. Formule pour nourrisson de la revendication 1, la formule pour nourrisson comprenant *B. longum* AH1206 dans la plage d'environ 1x10⁴ ufc à environ 1x10¹⁰ ufc par gramme de formule pour nourrisson.

3. Formule pour nourrisson de la revendication 1, dans laquelle *B. longum* AH 1206 est viable.

4. Formule pour nourrisson de la revendication 1, dans laquelle *B. longum* AH 1206 est non viable.

5. Formule pour nourrisson de la revendication 1, comprenant en outre un probiotique additionnel.

6. Formule pour nourrisson de la revendication 5, dans laquelle le probiotique additionnel comprend LGG.

7. Formule pour nourrisson de la revendication 1, comprenant en outre un prébiotique.

8. Formule pour nourrisson de la revendication 7, dans laquelle le prébiotique est choisi dans le groupe constitué des lactulose, gluco-oligosaccharide, inuline, polydextrose, galacto-oligosaccharide, fructo-oligosaccharide, isomalto-oligosaccharide, oligosaccharides de soja, lactosucrose, xylo-oligosaccharide, et gentio-oligosaccharides.

9. Formule pour nourrisson de la revendication 1, comprenant en outre au moins un LCPUFA.

10. Formule pour nourrisson de la revendication 1, comprenant en outre ARA et DHA.

11. Produit nutritionnel pour enfants comprenant une source de protéines, une source de lipides, une source de glucides, et *B. longum* AH1206*.*

12. *B.* /*ongum* AH 1206 pour utilisation dans un procédé pour réduire ou prévenir l'inflammation chez un nourrisson ou un enfant, le procédé comprenant l'administration de *B. longum* AH1206 au nourrisson ou enfant.

13. *B. longum* AH1206 pour utilisation dans le procédé de la revendication 12, la réduction ou la prévention de l'inflammation améliorant les allergies.

14. *B. longum* AH1206 pour utilisation dans le procédé de la revendication 12, la réduction ou la prévention de l'inflammation améliorant l'asthme.

15. *B. longum* AH1206 pour utilisation dans le procédé de la revendication 12, la réduction ou la prévention de l'inflammation améliorant une maladie choisie dans le groupe constitué de la diarrhée, les infections respiratoires, et l'otite moyenne.
